# EUROPEAN PATENT SPECIFICATION

(11) **EP 1 315 721 B1**
(45) Date of publication and mention of the grant of the patent: **25.10.2006**
(21) Application number: 01963244.7
(22) Date of filing: 05.09.2001
(51) Int. Cl.: C07D 417/12, A61K 31/4439, A61P 3/10

(54) **A THIAZOLIDINEDIONE DERIVATIVE AND ITS USE AS ANTIDIABETIC**
EIN THIAZOLIDINDIONDERIVAT UND SEINE VERWENDUNG ALS ANTIDIABETIKUM
DERIVE DE THIAZOLIDINEDIONE ET SON UTILISATION COMME ANTIDIABETIQUE

(30) Priority: 07.09.2000 GB 0021978
(43) Date of publication of application: 04.06.2003
(73) Proprietor: SMITHKLINE BEECHAM PLC, Brentford, Middlesex TW8 9GS (GB)
(72) Inventor: CRAIG, Andrew, Simon, c/o GlaxoSmithKline Pharma., Tonbridge, Kent TN11 9AN (GB); HO, Tim, Chien, Ting, c/o GlaxoSmithKline Pharma., Tonbridge, Kent TN11 9AN (GB)
(74) Representative: Rutter, Keith
(86) International application number: PCT/GB2001/003979
(87) International publication number: WO 2002/020517

(56) References cited:
- WO-A-00/00195
- WO-A-94/05659
- WO-A-99/31093
- WO-A-99/31094
- WO-A-99/31095

## Description

This invention relates to a novel pharmaceutical, to a process for the preparation of the pharmaceutical and to the use of the pharmaceutical in medicine.

European Patent Application, Publication Number 0,306,228 relates to certain thiazolidinedione derivatives disclosed as having hypoglycaemic and hypolipidaemic activity. The compound of example 30 af EP 0,306,228 is 5-[4-[2-(N-methyl-N-(2-pyridyl)amino)ethoxy]benzyl]thiazolidine-2,4-dione (hereinafter also referred to as "Compound (I)'').

International Patent Application, Publication Number WO94/05659 discloses certain salts of the compounds of EP 0,306,228. The preferred salt of WO94/05659 is the maleic acid salt.

WO99/31093, WO99/31094 and WO99/31095 disclose different forms of rosiglitazone maleate hydrate.

WO00/00195 generically discloses a large number of insulin sensitisers and their salts. Among the insulin sensitisers specifically identified are pioglitazone hydrochloride, troglitazone, rosiglitazone and 4-[4-[2-(5-methyl-2-phenyloxazol-4-yl)ethoxy]benzyl]isoxazolidin-3,5-dionc. The following salts of insulin sensitisers are referred to: salts with alkali metals such as sodium, potassium, *etc.* or alkaline earth metal such as calcium, magnesium, *etc*.; aluminium and ammonium salts; salts with organic bases including salts with trimethylamine, triethylamine, pyridine, picoline, ethanolamine, diethanolamine, triethanolamine, dicyclohexylamine, N,N-dibenzylethylenediamine, *etc.*; salts with inorganic acids including hydrochloric acid, hydrobromic acid, nitric acid, sulfuric acid, phosphoric acid, *etc*.; salts with organic acids including formic acid, acetic acid, trifluoroacetic acid, fumaric acid, oxalic acid, tartaric acid, maleic acid, citric acid, succinic acid, malic acid, methanesulfonic acid, benzenesulfonic acid, *p*-toluenesulfonic acid, *etc.*; salts with basic amino acids including arginine, lysine, ornithine, *etc*.; and salts with acidic amino acids including aspartic acid, glutamic acid, *etc.*

It has now been discovered that Compound (I) forms a novel fumarate salt (hereinafter also referred to as the "Fumarate") that is particularly stable and hence is suitable for bulk preparation and handling. The Fumarate also has a high melting point and possesses good bulk flow properties. The Fumarate is therefore surprisingly amenable to large scale pharmaceutical processing and especially to large scale milling.

The novel salt can be prepared by an efficient, economic and reproducible process particularly suited to large-scale preparation.

The novel Fumarate also has useful pharmaceutical properties and in particular it is indicated to be useful for the treatment and/or prophylaxis of diabetes mellitus, conditions associated with diabetes mellitus and certain complications thereof Accordingly, the present invention provides 5-[4-[2-(N-methyl-N-(2-pyridyl)amino)ethoxy]benzyl]thiazolidine-2,4-dione fumarate salt or a solvate thereof.

Suitably, the Fumarate is a mono-Fumarate salt as formed from a 1:1 molar ratio of Compound (I) and fumaric acid.

Mono Fumarate salts also optionally comprise another monovalent salting ion such as an alkali metal or ammonium cation, to provide a "mixed salt"

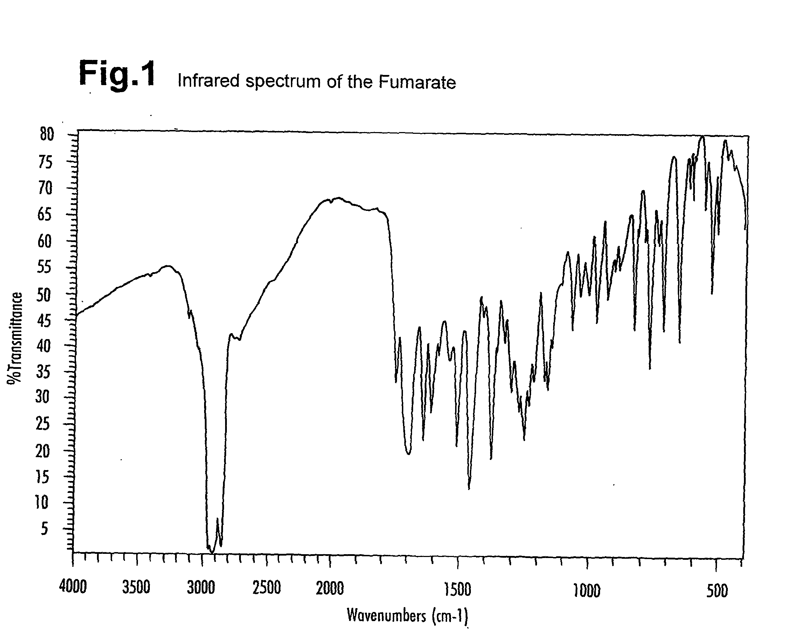

In one favoured aspect, the Fumarate provides an infrared spectrum substantially in accordance with Figure 1.

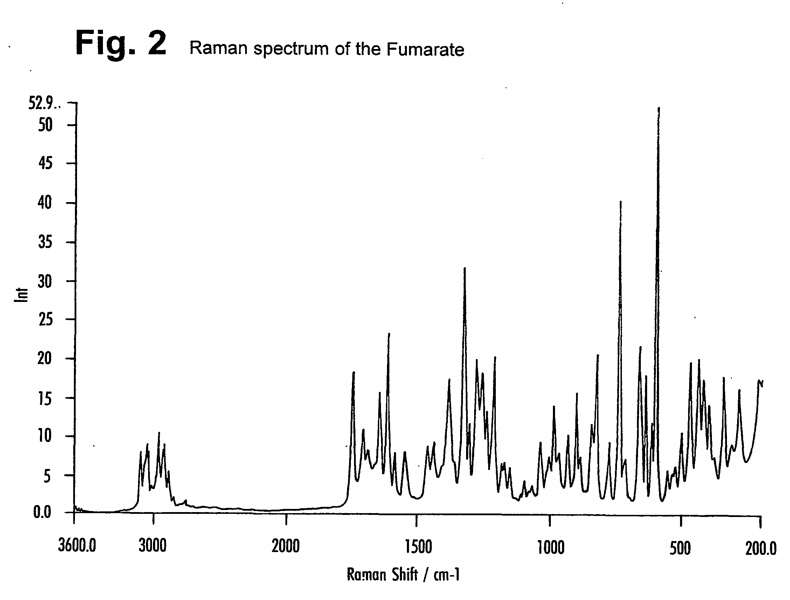

In one favoured aspect, the Fumarate provides a Raman spectrum substantially in accordance with Figure 2.

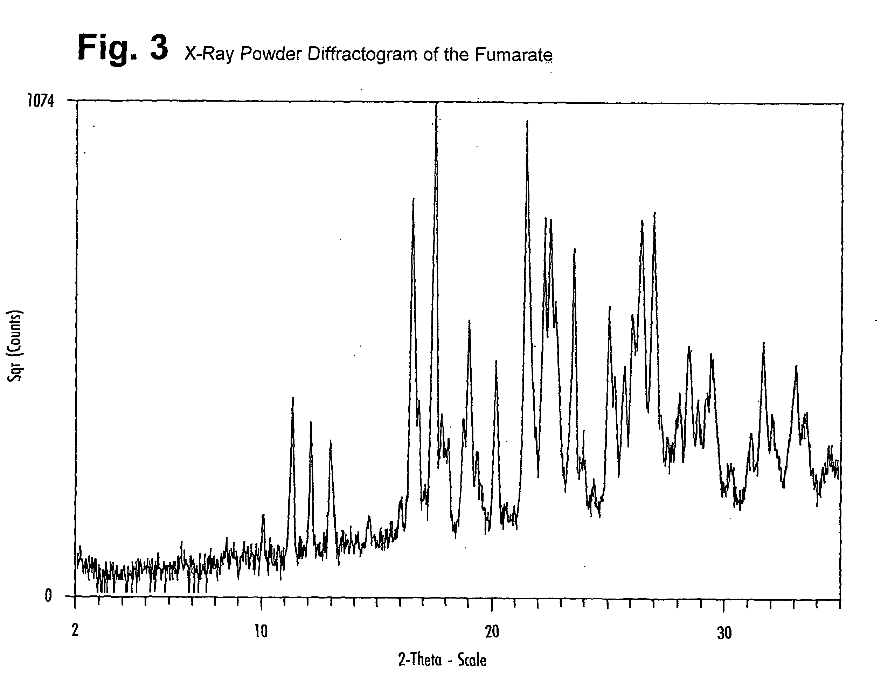

In one favoured aspect, the Fumarate provides an X-Ray powder diffraction pattern (XRPD) substantially in accordance with Table 1 or Figure 3.

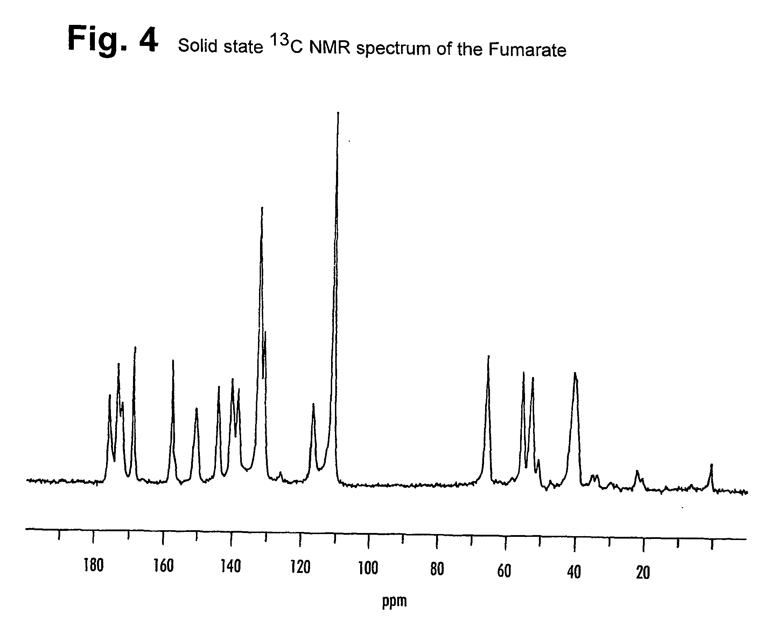

In one favoured aspect, the Fumarate provides a solid state ¹³C NMR spectrum substantially in accordance with Figure 4.

In a further favoured aspect, the Fumarate provides a melting point in the range of from 154 to 159 °C, such as 155 to 158°C, for example 157.4 °C .In a preferred aspect, the invention provides 5-[4-[2-(N-methyl-N-(2-pyridyl)amino)ethoxy]benzyl]thiazolidine-2,4-dione fumarate salt, characterised in that it provides:
(i) an infrared spectrum substantially in accordance with Figure 1; and
(ii) a Raman spectrum substantially in accordance with Figure 2; and
(iii) an X-Ray powder diffraction pattern (XRPD) substantially in accordance with Table 1 or Figure 3; and
(iv) a solid state ¹³C NMR spectrum substantially in accordance with Figure 4.

The present invention encompasses the Fumarate or a solvate thereof isolated in pure form or when admixed with other materials.

Thus in one aspect there is provided the Fumarate or a solvate thereof in isolated form.

In a further aspect there is provided the Fumarate or solvate thereof in a purified form.

In yet a further aspect there is provided the Fumarate or solvate thereof in crystalline form.

Also, the invention provides the Fumarate or solvate thereof in a solid pharmaceutically acceptable form, such as a solid dosage form, especially when adapted for oral administration.

Moreover, the invention also provides the Fumarate or solvate thereof in a pharmaceutically acceptable form, especially in bulk form, such form being particularly capable of being milled. The invention therefore also provides the Fumarate or solvate thereof in a milled form.

Furthermore, the invention provides the Fumarate or solvate thereof in a pharmaceutically acceptable form, especially in bulk form, such form having good flow properties, especially good bulk flow properties

A suitable solvate is a hydrate.

The invention also provides a process for preparing the Fumarate or a solvate thereof, characterised in that 5-[4-[2-(N-methyl-N-(2-pyridyl)amino)ethoxy]benzyl]thiazolidine-2,4-dione (Compound (I)) or a salt therof, preferably dispersed or dissolved in a suitable solvent, is reacted with a source of fumarate ion and thereafter, if required, a solvate of the resulting Fumarate is prepared; and the Fumarate or a solvate thereof is recovered.

Suitably, Compound (I) is used in the reaction, especially in a protonated form. Suitably, a salt of Compound (I) is used in the reaction..

Compound (I) is preferably present in a protonated form.

A suitable reaction solvent is a nitrile for example acetonitrile, an ether such as tetrahydrofuran, an alkanolsuch as methanol, or a hydrocarbon, such as toluene, a ketone, such as acetone, an ester, such as ethyl acetate, or a halogenated hydrocarbon such as dichloromethane, or water, or an organic acid such as acetic acid; or a mixture thereof.

Conveniently, the source of fumarate ion is fumaric acid. The fumaric acid is preferably added in solution but can be added as a solid. Suitable solvents for the fumaric acid include, water, a nitrile such as acetonitrile, tetrahydrofuran, or a lower alcohol such as methanol or ethanol, or a mixture of solvents. An alternative source of fumarate ion for a salt of Compound (I) is provided by a base salt of fumaric acid for example ammonium fumarate, or the fumaric acid salt of an amine, for example ethylamine or diethylamine.

The concentration of Compound (I) is preferably in the range 2 to 25% weight/volume, more preferably in the range 5 to 20%. The concentration of fumaric acid solutions are preferably in the range of 3 to 20% weight/volume.

The reaction is usually carried out at ambient temperature or at an elevated temperature, for example at the reflux temperature of the solvent, although any convenient temperature that provides the required product may be employed.

Solvates, such as hydrates, of the Fumarate are prepared according to conventional procedures.

Recovery of the required compound generally comprises crystallisation from an appropriate solvent, conveniently the reaction solvent, usually assisted by cooling. For example, the Fumarate may be crystallised from water or a nitrile such as acetonitrile or tetrahydrofuran or a mixture thereof. An improved yield of the salt can be obtained by evaporation of some or all of the solvent or by crystallisation at elevated temperature followed by controlled cooling, optionally in stages. Careful control of precipitation temperature and seeding may be used to improve the reproducibility of the product form.

Crystallisation can also be initiated by seeding with crystals of the Fumarate or a solvate thereof but this is not essential.

When the mono-Fumarate salt is a mixed salt comprising another monovalent salting ion, such as an alkali metal or ammonium cation, the said salt is conveniently formed by reacting the mono-Fumarate with a solution of the chosen monovalent salting ion, for example a metal or ammonium ion

Compound (I) is prepared according to known procedures, such as those disclosed in EP 0,306,228 and WO94/05659. The disclosures of EP 0,306,228 and WO94/05659 are incorporated herein by reference.

Fumaric acid is a commercially available compound.

When used herein the term "Tₒₙₛₑₜ" is generally determined by Differential Scanning Calorimetry and has a meaning generally understood in the art, as for example expressed in Pharmaceutical Thermal Analysis, Techniques and Applications", Ford and Timmins, 1989 as "The temperature corresponding to the intersection of the pre-transition baseline with the extrapolated leading edge of the transition".

When used herein in respect of certain compounds the term "good flow properties" is suitably characterised by the said compound having a Hausner ratio of less than or equal to 1.5, especially of less than or equal to 1.25.

"Hausner ratio" is an art accepted term.When used herein the term 'prophylaxis of conditions associated with diabetes mellitus' includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating, such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly provides the Fumarate or a solvate thereof for use as an active therapeutic substance.

More particularly, the present invention provides the Fumarate or a solvate thereof for use in the treatment and/or prophylaxis of diabetes mellitus, conditions associated with diabetes mellitus and certain complications thereof.

The Fumarate or a solvate thereof may be administered per se or, preferably, as a pharmaceutical composition also comprising a pharmaceutically acceptable carrier. Suitable methods for formulating the Fumarate or a solvate thereof are generally those disclosed for Compound (I) in the above mentioned publications.

Accordingly, the present invention also provides a pharmaceutical composition comprising the Fumarate or a solvate thereof and a pharmaceutically acceptable carrier therefor.

The Fumarate or a solvate thereof is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such, operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of Fumarate or a solvate thereof to a human or non-human mammal in need thereof.

The compositions are formulated according to conventional methods, such as those disclosed in standard reference texts, for example the British and US Pharmacopoeias, Remington's Pharmaceutical Sciences (Mack Publishing Co.), Martindale The Complete Drug Reference (London, The Pharmaceutical Press) and Harry's Cosmeticology (Leonard Hill Books).Conveniently, the active ingredient may be administered as a pharmaceutical composition hereinbefore defined, and this forms a particular aspect of the present invention.

In a further aspect the present invention provides the use of Fumarate or a solvate thereof for the manufacture of a medicament for the treatment and/or prophylaxis of diabetes mellitus, conditions associated with diabetes mellitus and certain complications thereof.

In the treatment and/or prophylaxis of diabetes mellitus, conditions associated with diabetes mellitus and certain complications thereof the Fumarate or a solvate thereof may be taken in amounts so as to provide Compound (I) in suitable doses, such as those disclosed in EP 0,306,228, WO94/05659 or WO98/55122.

No adverse toxicological effects are indicated in the above mentioned treatments for the compounds of the invention.

The following examples illustrate the invention but do not limit it in any way.

### EXAMPLES

### Example 1 5-[4-[2-(N-methyl-N-(2-pyridyl)amino)ethoxy]benzyl] thiazolidine-2,4-dione fumarate

A mixture of fumaric acid (0.65 g), acetonitrile (20 ml) and water (0.5 ml) was heated until a clear solution was observed, and was added to a stirred solution of 5-[4-[2-(N-methyl-N-(2-pyridyl)amino)ethoxy]benzyl]thiazolidine-2,4-dione (2.0 g) in acetonitrile (40 ml) at reflux. The reaction mixture was then cooled to 21°C with stirring. After filtration to remove a small amount of insoluble material the filtrate was maintained at 21°C for 72 hours. The product was collected by filtration, washed with acetonitrile (20 ml) and dried under vacuum to afford 5-[4-[2-(N-methyl-N-(2-pyridyl)amino)ethoxy]benzyl]thiazolidine-2,4-dione fumarate (0.83 g) as a crystalline solid.
¹H-NMR (d₆-DMSO): consistent with a 5-[4-[2-(N-methyl-N-(2-pyridyl)amino) ethoxy]benzyl]thiazolidine-2,4-dione fumarate (1:1 salt)

### Example 2 5-[4-[2-(N-methyl-N-(2-pyridyl)amino)ethoxy]benzyl] thiazolidine-2,4-dione fumarate

A mixture of fumaric acid (7.8 g), acetonitrile (50 ml) and water (40 ml) was heated until a clear solution was observed, and was added to a stirred solution of 5-[4-[2-(N-methyl-N-(2-pyridyl)amino)ethoxy]benzyl]thiazolidine-2,4-dione (12.0 g) in acetonitrile (200 ml) at reflux. The reaction mixture was stirred at reflux for 20 minutes, then cooled to 21°C over 1 hour and stirred for 16 hours at 21°C. The solid was collected by filtration, washed with acetonitrile (50 ml) then dried at 21°C under vacuum for 5 hours to afford 5-[4-[2-(N-methyl-N-(2-pyridyl)amino)ethoxy]benzyl]thiazolidine-2,4-dione fumarate (12.4 g) as a white crystalline solid.

### Example 3 5-[4-[2-(N-methyl-N-(2-pyridyl)amino)ethoxy]benzyl] thiazolidine-2,4-dione fumarate

A mixture of fumaric acid (26.0 g), acetonitrile (220 ml) and water (200 ml) was heated until a clear solution was observed, and was added to a stirred solution of 5-[4-[2-(N-methyl-N-(2-pyridyl)amino)ethoxy]benzyl]thiazolidine-2,4-dione (40.0 g) in acetonitrile (800 ml). The reaction mixture was heated at reflux for 5 minutes, then cooled to 21°C with stirring and maintained at this temperature for 48 hours. The solid was collected by filtration, washed with acetonitrile (100 ml) then dried under vacuum at 21°C for 5 hours to afford 5-[4-[2-(N-methyl-N-(2-pyridyl)amino)ethoxy]benzyl]thiazolidine-2,4-dione fumarate (34.7 g) as a white crystalline solid.

### Example 4 5-[4-[2-(N-methyl-N-(2-pyridyl)amino)ethoxy]benzyl] thiazolidine-2,4-dione fumarate

A hot clear solution of fumaric acid (1.3 g) in tetrahydrofuran (20 ml) was added to a clear stirring solution of 5-[4-[2-(N-methyl-N-(2-pyridyl)amino)ethoxy] benzyl]thiazolidine-2,4-dione (2.0 g) in tetrahydrofuran (20 ml) at reflux. The reaction mixture was cooled to 21°C with stirring until crystallisation was complete. The solid was collected by filtration, washed with tetrahydrofuran (30 ml) then dried under vacuum for 6.5 hours at 21°C to give 5-[4-[2-(N-methyl-N-(2-pyridyl)amino)ethoxy]benzyl]thiazolidine-2,4-dione fumarate as a crystalline solid (1.31g).

### Characterising data recorded for the product of Example 1:

The infrared absorption spectrum of a mineral oil dispersion of the product was obtained using a Nicolet 710 FT-IR spectrometer at 2 cm⁻¹ resolution (Figure 1). Data were digitised at 1 cm⁻¹ intervals. Bands were observed at: 2925, 2854, 1749, 1699, 1643, 1611, 1513, 1460, 1377, 1328, 1302, 1250, 1216, 1174, 1161, 1067, 1037, 1002, 973, 928, 885, 826, 767, 735, 714, 653, 617, 603, 557, 527, 507 cm⁻¹.

The infrared spectrum of the solid product was recorded using a Perkin-Elmer Spectrum One FT-IR spectrometer fitted with a universal ATR accessory. Bands were observed at: 2932, 2765, 1749, 1693, 1642, 1611, 1541, 1513, 1468, 1411, 1382, 1357, 1328, 1303, 1270, 1250, 1235, 1216, 1174, 1161, 1146, 1066, 1036, 1002, 972, 928, 903, 884, 825, 786, 766, 736, 714 cm⁻¹.

The Raman spectrum of the product (Figure 2) was recorded with the sample in a glass vial using a Perkin-Elmer 2000R FT-Raman spectrometer, at 4 cm⁻¹ resolution with excitation from a Nd:YAG laser (1064 nm) with a power output of 400mW. Bands were observed at: 3099, 3055, 2965, 2917, 1747, 1707, 1645, 1613, 1585, 1547, 1438, 1382, 1327, 1305, 1278, 1257, 1211, 1169, 1036, 985, 934, 902, 826, 779, 742, 664, 639, 617, 604, 504, 472, 441, 423, 401, 346, 287 cm⁻¹.

The X-Ray Powder Diffractogram pattern of the product (Figure 3) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds.Characteristic XRPD angles and relative intensities are recorded in Table 1.

**Table 1**

| **Angle** | **Rel. Intensity** |
|---|---|
| **2-Theta** ° | % |
| 10.1 | 2.8 |
| 11.3 | 16.3 |
| 12.1 | 12.4 |
| 13.0 | 10 |
| 14.6 | 2.7 |
| 16.0 | 3.7 |
| 16.5 | 65.5 |
| 16.8 | 15.9 |
| 17.5 | 100 |
| 17.8 | 12.8 |
| 18.1 | 10.4 |
| 18.8 | 13 |
| 19.0 | 31.8 |
| 19.4 | 8.8 |
| 20.2 | 23.1 |
| 21.5 | 93.6 |
| 22.3 | 59.7 |
| 22.6 | 58.8 |
| 22.8 | 30.8 |
| 23.6 | 50.2 |
| 24.0 | 11.4 |
| 24.5 | 5.9 |
| 25.1 | 35.1 |
| 25.4 | 20.1 |
| 25.8 | 21.9 |
| 26.2 | 33 |
| 26.5 | 58.9 |
| 27.1 | 61.3 |
| 28.2 | 17.5 |
| 28.6 | 26.2 |
| 29.0 | 16 |
| 29.3 | 17.1 |
| 29.6 | 24.4 |
| 30.4 | 7.4 |
| 31.3 | 11 |
| 31.8 | 27 |
| 32.1 | 14 |
| 33.2 | 20.2 |
| 33.6 | 14 |
| 34.6 | 8.4 |

The solid-state NMR spectrum of the product (Figure 4) was recorded on a Bruker AMX360 instrument operating at 90.55 MHz: The solid was packed into a 4 mm zirconia MAS rotor fitted with a Kel-F cap and rotor spun at ca. 10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 40.7, 52.9, 55.5, 66.0, 110.9, 116.7, 131.1, 132.4, 138.5, 140.3, 144.3, 150.6, 157.7, 168.9, 173.4, 175.8 ppm.

### Properties of the Fumarate, recorded for the product of Example 3

### Solid State Stability of the Fumarate

The solid state stability of the drug substance was determined by storing approximately 1.0 g of the material in a glass bottle at a) 40°C /75% Relative Humidity (RH), open exposure, for 1 month and b) at 50°C, closed, for 1 month. The material was assayed by HPLC for final content and degradation products in both cases.
a) 40°C /75% RH: No significant degradation observed (HPLC assay 100% initial).
b) 50°C: No significant degradation observed (HPLC assay 99% initial).

### Flow Properties of the Fumarate

The ratio between the bulk density and the tapped bulk density (Hausner Ratio) of the Fumarate was determined using standard methods ("Pharmaceutics - The Science of Dosage Form Design", editor M. Aulton, 1988, published by:Churchill Livingstone).
Hausner Ratio: 1.0

### Melting Point of the Fumarate

The melting point of the Fumarate was determined according to the method described in the U.S. Pharmacopoeia, USP 23, 1995, <741> "Melting range or temperature, Procedure for Class Ia", using a Buchi 545 melting point instrument.
Melting Point: 157.4°C

### Tₒₙₛₑₜ of the Fumarate

The Tₒₙₛₑₜ of the drug substance was determined by Differential Scanning Calorimetry using a Perkin-Elmer DSC7 apparatus.
Tₒₙₛₑₜ (10°C/minute, closed pan): 156 °C

## Claims

1. A compound 5-[4-[2-(N-methyl-N-(2-pyridyl)amino)ethoxy]benzyl]thiazolidine-2,4-dione fumarate salt or a solvate thereof.

2. A compound according to claim 1, **characterised in that** it provides:
(i) an infrared spectrum substantially in accordance with Figure 1; and
(ii) a Raman spectrum substantially in accordance with Figure 2; and
(iii) an X-Ray powder diffraction pattern (XRPD) substantially in accordance with Table 1 or Figure 3; and
(iv) a solid state ¹³C NMR spectrum substantially in accordance with Figure 4.

3. A compound according to claim 1, **characterised in that** it provides two or more of:
(i) an infrared spectrum substantially in accordance with Figure 1;
(ii) a Raman spectrum substantially in accordance with Figure 2;
(iii) an X-Ray powder diffraction pattern (XRPD) substantially in accordance with Table 1 or Figure 3;
(iv) a solid state ¹³C NMR spectrum substantially in accordance with Figure 4; and
(v) a melting point in the range of from 154 to 159 °C.

4. A compound according to any one of claims 1 to 3, in purified form.

5. A compound according to any one of claims 1 to 3, in a solid dosage form.

6. A compound according to any one of claims 1 to 3, in a pharmaceutically acceptable form capable of being milled or in milled form

7. A compound according to any one of claims 1 to 3, in a pharmaceutically acceptable form and having good flow properties.

8. A process for preparing the fumarate or a solvate thereof, **characterised in that** 5-[4-[2-(N-methyl-N-(2-pyridyl)amino)ethoxy]benzyl]thiazolidine-2,4-dione (Compound (I)) or a salt thereof, preferably dispersed or dissolved in a suitable solvent, is reacted with a source of fumarate ion and thereafter, if required, a solvate of the resulting fumarate is prepared; and the fumarate or a solvate thereof is recovered.

9. A pharmaceutical composition comprising 5-[4-[2-(N-methyl-N-(2-pyridyl)amino)ethoxy]benzyl]thiazolidine-2,4-dione fumarate or a solvate thereof: and a pharmaceutical acceptable carrier therefor.

10. A compound 5-[4-[2-(N-methyl-N-(2-pyridyl)amino)ethoxy]benzyl]thiazolidine-2,4-dione fumarate or a solvate thereof for use as an active therapeutic substance.

11. A use of 5-[4-[2-(N methyl-N-(2-pyridyl)amino)ethoxy]benzyl]thiazolidine-2,4-dione fumarate or a solvate thereof, for the manufacture of a medicament for the treatment and/or prophylaxis of diabetes mellitus, conditions associated with diabetes mellitus and certain complications thereof.

## Patentansprüche

1. Verbindung 5-[4-[2-(N-Methyl-N-(2-pyridyl)amino)ethoxy]benzyl]thiazolidin-2,4-dion-fumaratsalz oder ein Solvat davon.

2. Verbindung gemäß Anspruch 1, **dadurch gekennzeichnet, dass** sie bereitstellt:
(i) ein Infrarotspektrum im wesentlichen gemäß Figur 1; und
(ii) ein Ramanspektrum im wesentlichen gemäß Figur 2; und
(iii) ein Röntgenpulverbeugungsmuster (XRPD) im wesentlichen gemäß Tabelle 1 oder Figur 3; und
(iv) ein Festphasen-¹³C NMR-Spektrum im wesentlichen gemäß Figur 4.

3. Verbindung gemäß Anspruch 1, **dadurch gekennzeichnet, dass** sie zwei oder mehr aus
(i) einem Infrarotspektrum im wesentlichen gemäß Figur 1;
(ii) einem Ramanspektrum im wesentlichen gemäß Figur 2;
(iii) einem Röntgenpulverbeugungsmuster (XRPD) im wesentlichen gemäß Tabelle 1 oder Figur 3;
(iv) ein Festphasen-¹³C NMR-Spektrum im wesentlichen gemäß Figur 4; und
(v) einem Schmelzpunkt im Bereich von 154 bis 159°C bereitstellt.

4. Verbindung gemäß einem der Ansprüche 1 bis 3 in gereinigter Form.

5. Verbindung gemäß einem der Ansprüche 1 bis 3 in einer festen Dosierungsform.

6. Verbindung gemäß einem der Ansprüche 1 bis 3 in einer pharmazeutisch verträglichen Form, die gemahlen werden kann, oder in gemahlener Form.

7. Verbindung gemäß einem der Ansprüche 1 bis 3 in einer pharmazeutisch verträglichen Form und mit guten Fließeigenschaften.

8. Verfahren zur Herstellung des Fumarats oder eines Solvats davon, **dadurch gekennzeichnet, dass** 5-[4-[2-(N-Methyl-N-(2-pyridyl)amino)ethoxy]benzyl]thiazolidin-2,4-dion (Verbindung (I)) oder ein Salz davon, vorzugsweise in einem geeigneten Lösungsmittel dispergiert oder gelöst, mit einer Quelle von Fumarationen umgesetzt wird und anschließend, falls erwünscht, ein Solvat des erhaltenen Fumarats hergestellt wird; und das Fumarat oder ein Solvat davon gewonnen wird.

9. Arzneimittel, umfassend 5-[4-[2-(N-Methyl-N-(2-pyridyl)amino)ethoxy]benzyl]-thiazolidin-2,4-dionfumarat oder ein Solvat davon und einen pharmazeutisch verträglichen Träger dafür.

10. Verbindung 5-[4-[2-(N-Methyl-N-(2-pyridyl)amino)ethoxy]benzyl]thiazolidin-2,4-dion-fumarat oder ein Solvat davon, zur Verwendung als einen therapeutischen Wirkstoff.

11. Verwendung des 5-[4-[2-(N-Methyl-N-(2-pyridyl)amino)ethoxy]benzyl]thiazolidin-2,4-dionfumarats oder eines Solvats davon, zur Herstellung eines Medikaments zur Behandlung und/oder Vorbeugung von Diabetes mellitus, mit Diabetes mellitus in Verbindung stehenden Zuständen und bestimmten Komplikationen davon.

## Revendications

1. Composé qui est le sel fumarate de 5-[4-[2-(N-méthyl-N-(2-pyridyl)amino)éthoxy]benzyl]thiazolidine-z,4-dione ou un de ses produits de solvatation.

2. Composé suivant la revendication 1, **caractérisé en ce qu'**il présente :
(i) un spectre infrarouge substantiellement suivant la figure 1 ; et
(ii) un spectre Raman substantiellement suivant la figure 2 ; et
(iii) un diagramme de diffraction des rayons X sur poudre (XRPD) substantiellement suivant le tableau 1 ou la figure 3 ; et
(iv) un spectre de ¹³CRMN à l'état solide substantiellement suivant la figure 4.

3. Composé suivant la revendication 1, **caractérisé en ce qu'**il présente deux ou plus de deux de :
(i) un spectre infrarouge substantiellement suivant la figure 1 ;
(ii) un spectre Raman substantiellement suivant la figure 2 ;
(iii) un diagramme de diffraction des rayons X sur poudre (XRPD) substantiellement suivant le tableau 1 ou la figure 3 ;
(iv) un spectre de ¹³CRMN à l'état solide substantiellement suivant la figure 4 ; et
(v) un point de fusion compris dans l'intervalle de 154 à 159°C.

4. Composé suivant l'une quelconque des revendications 1 à 3, sous forme purifiée.

5. Composé suivant l'une quelconque des revendications 1 à 3, sous une forme posologique solide.

6. Composé suivant l'une quelconque des revendications 1 à 3, sous une forme pharmaceutiquement acceptable pouvant être broyée ou bien sous une forme broyée.

7. Composé suivant l'une quelconque des revendications 1 à 3, sous une forme pharmaceutiquement acceptable et ayant de bonnes propriétés d'écoulement.

8. Procédé pour la préparation du fumarate ou d'un des ses produits de solvatation, **caractérisé en ce que** de la 5-[4-[2-(N-méthyl-N-(2-pyridyl)amine)éthoxy]benzyl]thiazolidine-2,4-dione (composé (I) ou un de ses sels, de préférence à l'état dispersé ou dissous dans un solvant convenable, est amenée à réagir avec une source d'ions fumarate et ensuite, si besoin, un produit de solvatation du fumarate résultant est préparé ; et le fumarate ou un de ses produits de solvatation est recueilli.

9. Composition pharmaceutique comprenant du fumarate de 5-[4-[2-(N-méthyl-N-(2-pyridyl)amino)éthoxy]benzyl] thiazolidine-2,4-dione ou un de ses produits de solvatation, et un support pharmaceutiquement acceptable à cette fin.

10. Composé qui est le fumarate de 5-[4-[2-(N-méthyl-N-(2-pyridyl)amino)éthoxy]benzyl]thiazolidine-2,4-dione ou un de ses produits de solvatation, destiné à être utilisé comme substance thérapeutique active.

11. Utilisation du fumarate de 5-[4-[2-(N-méthyl-N-(2-pyridyl)amino)éthoxy]benzyl]thiazolidine-2,4-dione ou d'un de ses produits de solvatation, pour la production d'un médicament destiné au traitement et/ou à la prophylaxie du diabète sucré, d'affections associées au diabète sucré et de certaines de leurs complications.
